# FASCICULE DE BREVET EUROPEEN

(11) **EP 2 310 383 B1**
(45) Date de publication et mention de la délivrance du brevet: **30.11.2011**
(21) Numéro de dépôt: 09761938.1
(22) Date de dépôt: 12.06.2009
(51) Int. Cl.: C07D 401/14, A61K 31/496, A61P 35/00, A61P 29/00, A61P 25/00

(54) **DERIVES DE 2-OXO-ALKYL-1-PIPERAZIN-2-ONE, LEUR PREPARATION ET LEUR APPLICATION EN THERAPEUTIQUE**
DERIVATE VON 2-OXOALKYL-1-PIPERAZIN-2-ON, VERFAHREN ZU DEREN HERSTELLUNG UND DEREN THERAPEUTISCHE VERWENDUNG
DERIVATIVES OF 2-OXO-ALKYL-1-PIPERAZIN-2-ONE, PREPARATION METHOD THEREOF AND THERAPEUTIC USE OF SAME

(30) Priorité: 13.06.2008 FR 0803298
(43) Date de publication de la demande: 20.04.2011
(73) Titulaire: SANOFI, 75013 Paris (FR)
(72) Inventeur: BARONI, Marco, F-75013 Paris (FR); BONO, Françoise, F-75013 Paris (FR); DELBARY-GOSSART, Sandrine, F-75013 Paris (FR)
(74) Mandataire: Veinante, Aude
(86) Numéro de dépôt international: PCT/FR2009/051117
(87) Numéro de publication internationale: WO 2009/150387

(56) Documents cités:
- WO-A-97/28140
- WO-A-99/01423
- WO-A-03/104225
- WO-A-2005/054227
- WO-A-2005/054229

## Description

La présente invention a pour objet des dérivés de dérivés de 4-{2-[phényl-3,6-dihydro-pyridin-1-yl]-2-oxo-alkyl}-1-pipérazin-2-one et 4-{2-[phényl-2,5-dihydro-pyrrol-1-yl]-2-oxo-alkyl}-1-pipérazin-2-one, leur préparation et leur application en thérapeutique.

Les composés selon la présente invention présentent une affinité pour le récepteur p75^{NTR} des neurotrophines.

Les neurotrophines appartiennent à une famille de protéines ayant notamment pour effet biologique la survie et la différenciation cellulaires.

Le récepteur p75^{NTR}, récepteur de toutes les neurotrophines, est une glycoprotéine transmembranaire de la famille du récepteur du facteur de nécrose tumorale (TNF, de l'anglais Tumor Necrosis Factor) (W. J. Friedman et L. A. Greene, Exp. Cell. Res., 1999,253, 131-142). Le récepteur p75^{NTR} est exprimé dans plusieurs types cellulaires, et plusieurs fonctions biologiques lui sont attribuées : d'une part, la modulation de l'affinité des neurotrophines pour les récepteurs tyrosine kinase (trk) ; d'autre part, en l'absence de trk, une induction d'un signal de mort cellulaire par apoptose. Par ailleurs, les précurseurs des neurotrophines, les proneurotrophines sont capables de se fixer sur p75^{NTR} avec une haute affinité, et sont considérés comme de puissants inducteurs de l'apoptose dépendant de p75^{NTR} dans les neurones et certaines lignées cellulaires.

Au niveau du système nerveux central, de nombreux travaux montrent que l'apoptose intervient dans plusieurs pathologies comme la sclérose latérale amyotrophique, la sclérose en plaques, les maladies d'Alzheimer, de Parkinson et d'Huntington et les maladies à prion. P75^{NTR} est également connu pour être surexprimé dans différents types de maladies neurodégénératives comme la maladie d'Alzheimer et la sclérose latérale amyotrophique (ALS) (Longo F. M. et al., Curr. Alzheimer Res. 2007;4: 503-506; Lowry K.S. et al., Amyotroph. Lateral. Scler. Other. Motor. Neuron. Disord. 2001; 2:127-34).

Des résultats suggèrent que p75^{NTR} peut jouer un rôle prépondérant dans les mécanismes conduisant à la mort neuronale par apoptose post-ischémie (P. P. Roux et al., J. Neurosci., 1999, 19, 6887- 6896).

Des résultats (V. Della-Bianca et al., J. Biol. Chem., 2001, 276 : 38929-33), (S. Rabizadeh et al., Proc. Natl. Acad. Sci. USA, 1994,91, 10703-10706) supportent l'hypothèse selon laquelle p75^{NTR} jouerait un rôle important dans la mort neuronale induite par la protéine prion infectieuse (encéphalopathie spongiforme transmissible) ou par la protéine beta Amyloide (maladie d'Alzheimer).

Le récepteur p75^{NTR} est également associé au récepteur Nogo et impliqué dans la signalisation des effets inhibiteurs de ces protéines de la myéline vis-à-vis de la croissance axonale. De ce fait, le récepteur p75^{NTR} joue un rôle majeur dans la régulation de la plasticité neuronale et dans les interactions neurones-glie et représente ainsi une cible thérapeutique de choix pour promouvoir la régénération nerveuse.

Au-delà du système nerveux et des maladies neurodégénératives, il a été suggéré que p75^{NTR} pouvait jouer un rôle dans des maladies cardiovasculaires telles que l'athérosclérose et l'ischémie du myocarde (M. L. Bochaton-Pialat et al., Am. J. Pathol., 1995,146, 1-6 ; H. Perlman, Circulation, 1997,95, 981-987). Des travaux récents montrent une augmentation de l'expression de p75^{NTR} et des neurotrophines, et une apoptose massive dans des lésions d'athérosclérose.

Plusieurs études suggèrent également que p75^{NTR} est un médiateur de l'inflammation (Rihl M. et al., Ann. Rheum. Dis. 2005; 64(11):1542-9 ; Raychaudhuri S.P. et al., Prog. Brain. Res. 2004;146: 433-7, Tokuoka S. et al., Br. J. Pharmacol. 2001, 134: 1580-1586).

P75^{NTR} joue également un rôle critique dans la biologie tumorale.

De nombreux composés sont connus pour interagir avec le système trkA/NGF/p75^{NTR} ou pour posséder une activité de type NGF (facteur de croissance neuronale, de l'anglais nerve growth factor). Ainsi la demande de brevet WO 00/59893 décrit des dérivés de pyrimidines substituées qui présentent une activité de type NGF et/ou qui augmentent l'activité du NGF sur les cellules PC12.

La demande de brevet WO03/104225 décrit des composés qui présentent une affinité pour les récepteurs P75^{NTR}. Ces composés sont fortement métabolisés et présentent de forts pourcentages d'inhibition du gène hERG (the human Ether à go-go Related Gene).

Le gène hERG code pour la protéine Kᵥ11.1. d'un canal ionique à potassium. Cette protéine est connue du fait de sa contribution à l'activité électrique du coeur. Quand la capacité du canal à conduire le courant électrique à travers la membrane cellulaire est inhibée par l'action de médicaments, il peut aboutir à un trouble potentiellement fatal appelé le QT syndrome. Un certain nombre de médicaments ont inhibé cette protéine, créant un risque concomitant de mort subite comme un effet secondaire indésirable. Ceci a fait de l'inhibition hERG une question centrale tant dans la régulation des médicaments que dans leur développement (Sanguinetti MC, Tristani-Firouzi M (March 2006). "hERG potassium channels and cardiac arrhythmia". Nature 44.0 (7083): 463-9).

La présente invention a pour objet des nouveaux composés qui présentent une affinité pour les récepteurs P75^{NTR} et qui ne présentent pas les inconvénients de forte métabolisation et de forte inhibition hERG des composés de l'art antérieur. Elle présente donc un avantage pour le développement de nouveaux médicaments.

La présente invention a pour objet les composés répondant à la formule (I) : 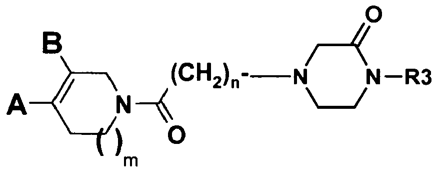 dans laquelle :
- m représente 0 ou 1 ;
- A représente : 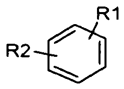
et B représente un atome d'hydrogène
ou
A représente un atome d'hydrogène et B représente : 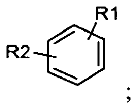 -R1 et R2, identiques ou différents, représentent indépendamment un atome d'hydrogène ou d'halogène, un groupe C1-4 alkyle, C1-4 fluoroalkyle, C1-2 perfluoroalkyle, C1-4 alcoxy ou un groupe trifluorométhoxy;
- n est 1 ou 2 ;
- R3 représente un groupe de formule: 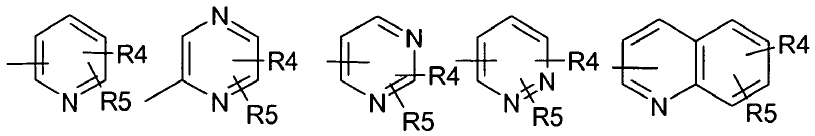 où R 4 et R5, identiques ou différents, sont situés sur des positions quelconques disponibles, et représentent indépendamment un atome d'hydrogène ou d'halogène, un hydroxyle, un groupe C1-4 alkyle, C1-4 fluoroalkyle, C1-2 perfluoroalkyle, C1-4 alcoxy, un groupe trifluorométhoxy, un groupe cyano, un groupe COOH, COOalkyle, CONH2, CONR6R7 ou NHCOR ;
- R,R6 et R7 représentent un C1-C6 alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par :
- un atome d'halogène : un fluor, un chlore, un brome ou un iode ;
- un groupe alkyle : un groupe aliphatique saturé linéaire ou ramifié. A titre d'exemples, on peut citer un groupe C1-4 alkyle qui peut représenter un méthyle, éthyle, propyle, isopropyle, butyle, isobutyle, tertbutyle ;
- un groupe fluoroalkyle : un groupe alkyle dont un ou plusieurs atomes d'hydrogène ont été substitués par un atome de fluor ;
- un groupe perfluoroalkyle : un groupe alkyle dont tous les atomes d'hydrogène ont été substitués par un atome de fluor ;
- un groupe alcoxy : un radical -O-alkyle où le groupe alkyle est tel que précédemment défini.

Parmi les composés de formule (I) objets de l'invention, un autre groupe de composés est constitué par ceux pour lesquels R4 et R5, identiques ou différents, sont situés sur des positions quelconques disponibles, et représentent indépendamment CONH2, CONR6R7 ou NHCOR, R, R6 et R7 étant définis comme précédemment ;
à l'état de bases ou de sels d'addition à des acides.

Parmi les composés de formule (I) objets de l'invention, un autre groupe de composés est constitué par les composés de formule (I) dans laquelle :
- m représente 1 ;
- A représente : 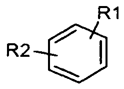 et B représente un atome d'hydrogène ;
- R1 et R2, identiques ou différents, représentent indépendamment un atome d'hydrogène ou d'halogène, un groupe C1-4 alkyle, C1-4 fluoroalkyle, C1-2 perfluoroalkyle, C1-4 alcoxy ou un groupe trifluorométhoxy;
- n est 1 ou 2 ;
- R3 représente un groupe de formule: 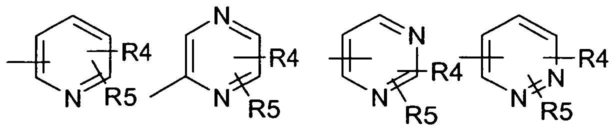 où R 4 et R5, identiques ou différents, sont situés sur des positions quelconques disponibles, et représentent indépendamment un atome d'hydrogène ou d'halogène, un hydroxyle, un groupe C1-4 alkyle, C1-4 fluoroalkyle, C1-2 perfluoroalkyle, C1-4 alcoxy, un groupe trifluorométhoxy, un groupe cyano, un groupe COOH ou COOalkyle ;
   à l'état de bases ou de sels d'addition à des acides.

Parmi les composés de formule (I) objets de l'invention, un autre groupe de composés est constitué par ceux pour lesquels R1 est différent de H, R2 étant défini comme précédemment ;
à l'état de bases ou de sels d'addition à des acides.

Parmi les composés de formule (I) objets de l'invention, un autre groupe de composés est constitué par les composés pour lesquels :
R1 est en position -2-, -3- ou -4- du phényle et représente un atome d'halogène ou plus particulièrement de chlore, un radical CF3 et R2 représente un hydrogène ou un 3- ou 4-halogène plus particulièrement un 3- ou 4-CI ; ou bien R1 est en position 2-, 3- ou 4- et représente un atome de chlore ou un radical CF3 et R2 représente un hydrogène ; ou bien R1 est en position 3- du phényle et représente un radical CF3 et R2 est en position 4- du phényle et représente un atome de chlore ; ou bien R1 est en position 2- du phényle et représente un atome de chlore et R2 est en position 3- du phényle et représente un atome de chlore; et/ou
R3 représente un 2-pyridynyle ou un 2 -pyrimidinyle, chacun substitué par R4 et R5 tels que définis ci-avant ; et/ou
n est = 1 ;
à l'état de bases ou de sels d'addition à des acides.

Parmi les composés de ce dernier groupe, on peut citer les composés de formule (I) pour lesquels :
R1 représente le 3-CF3 ;
R2 représente le 4-Chloro ;
R3 représente un résidu 2-pyridyl substitué en 5 par un CF3 ; et
n est = 1 ;
à l'état de bases ou de sels d'addition à des acides.

Parmi les composés de formule (I) objets de l'invention, on peut notamment citer les composés suivants :
- Composé n°1 :4-{2-[4-(4-chloro-3-trifluorométhyl-phényl)-3,6-dihydro-2*H-*pyridin-1-yl]-2-oxo-éthyl}-1-(5-trifluorométhyl-pyridin-2-yl)-pipérazin-2-one ;
- Composé n°2 :4-{2-[4-(4-chloro-3-trifluorométhyl-phényl)-3,6-dihydro-2*H-*pyridin-1-yl]-2-oxo-éthyl}-1-(5-méthyl-pyridin-2-yl)-pipérazin-2-one ;
- Composé n°3 : 4-{2-[4-(4-chloro-phényl)-3,6-dihydro-2*H*-pyridin-1-yl]-2-oxo-éthyl}-1-(5-trifluorométhyl-pyridin-2-yl)-pipérazin-2-one ;

- Composé n°4 : 4-{2-Oxo-2-[4-(3-trifluorométhyl-phényl)-3,6-dihydro-2H-pyridin-1-yl]-éthyl}-1-pyridin-2-yl-pipérazin-2-one;
- Composé n°5 : 4-{2-[4-(4-chloro-3-trifluorométhyl-phényl)-3,6-dihydro-2*H-*pyridin-1-yl]-2-oxo-éthyl}-1-pyridin-2-yl-pipérazin-2-one ;
- Composé n°6 : 4-{2-[4-(4-chloro-phényl)-3,6-dihydro-2*H*-pyridin-1-yl]-2-oxo-éthyl}-1-pyridin-2-yl-pipérazin-2-one ;
- Composé n°7 :4-{2-[4-(2,3-dichloro-phényl)-3,6-dihydro-2*H*-pyridin-1-yl]-2-oxo-éthyl}-1-(5-trifluorométhyl-pyridin-2-yl)-pipérazin-2-one ;
- Composé n°8 : 4-{2-[4-(4-chloro-phényl)-3,6-dihydro-2*H*-pyridin-1-yl]-2-oxo-éthyl}-1-(6-chloro-pyridin-2-yl)-pipérazin-2-one ;
- Composé n°9 : 4-{2-[4-(3-chloro-phényl)-3,6-dihydro-2*H*-pyridin-1-yl]-2-oxo-éthyl}-1-(5-trifluorométhyl -pyridin-2-yl)-pipérazin-2-one ;
- Composé n°10 : 4-{2-[4-(4-trifluorométhyl-phényl)-3,6-dihydro-2*H*-pyridin-1-yl]-2-oxo-éthyl}-1-(5-trifluorométhyl -pyridin-2-yl)-pipérazin-2-one ;
- Composé n°11 : 4-{2-[4-(3-trifluorométhyl-phényl)-3,6-dihydro-2*H*-pyridin-1-yl]-2-oxo-éthyl}-1-(5-trifluorométhyl-pyridin-2-yl)-pipérazin-2-one ;
- Composé n°12 : 4-{2-[4-(4-chloro-3-trifluorométhyl-phényl)-3,6-dihydro-2*H-*pyridin-1-yl]-2-oxo-éthyl}-1-pyridin-3-yl-pipérazin-2-one ;
- Composé n°13 : 1-(6-Chloro-pyridin-3-yl)-4-{2-[4-(4-chloro-3-trifluorométhyl-phényl)-3,6-dihydro-2H-pyridin-1-yl]-2-oxo-éthyl}-pipérazin-2-one;
- Composé n°14 : 4-{2-Oxo-2-[5-(3-trifluorométhyl-phényl)-3,6-dihydro-2H-pyridin-1-yl]-éthyl}-1-(5-trifluorométhyl-pyridin-2-yl)-pipérazin-2-one;
- Composé n°15 : 4-{2-Oxo-2-[4-(3-trifluorométhoxyl-phényl)-3,6-dihydro-2H-pyridin-1-yl]-éthyl}-1-pyridin-2-yl-pipérazin-2-one;
- Composé n°16 : 4-{2-[4-(4-chloro-3-trifluorométhyl-phényl)-2,5-dihydro-pyrrol -1-yl]-2-oxo-éthyl}-1-(5-trifluorométhyl-pyridin-2-yl)-pipérazin-2-one ;
- Composé n°17 : 4-{2-[4-(3,5bis-trifluorométhyl-phényl)-3,6-dihydro-2H-pyridin 1-yl]-2-oxo-éthyl}-1-(5-trifluorométhyl-pyridin-2-yl)-pipérazin-2-one ;
- Composé n°18 : 4-{2-[4-(3-méthyl-phényl)-3,6-dihydro-2H-pyridin -1-yl]-2-oxo-éthyl}-1-(5-trifluorométhyl-pyridin-2-yl)-pipérazin-2-one ;
- Composé n°19 : 4-{2-[4-phényl-3,6-dihydro-2H-pyridin -1-yl]-2-oxo-éthyl}-1-(5-trifluorométhyl-pyridin-2-yl)-pipérazin-2-one ;
- Composé n°20 : 4-{2-Oxo-2-[5-(2,3-dichloro-phényl)-3,6-dihydro-2H-pyridin-1-yl]-éthyl}-1-(5-trifluorométhyl-pyridin-2-yl)-pipérazin-2-one;
- Composé n°21 : 4-{2-Oxo-2-[5-(3-méthoxy-phényl)-3,6-dihydro-2H-pyridin-1-yl]-éthyl}-1-(5-trifluorométhyl-pyridin-2-yl)-pipérazin-2-one;
   à l'état de bases ou de sels d'addition à des acides.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York).

Conformément à l'invention, on peut préparer les composés de formule générale (I) selon le procédé qui suit.

### Schéma 1 :

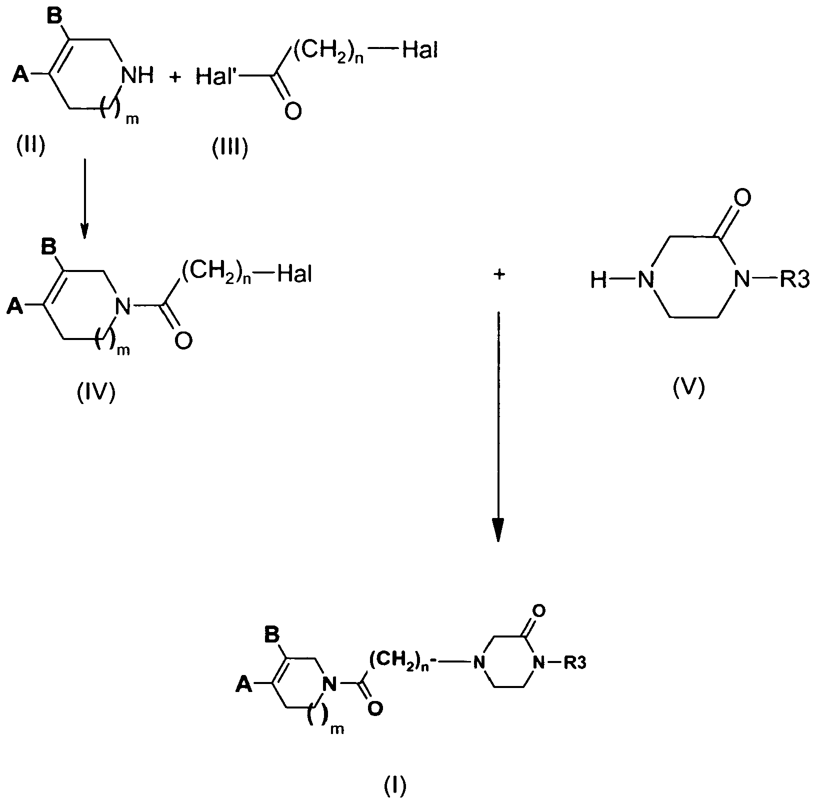

Les composés de formule (I) dans laquelle A, B, m, n, R3 sont tels que définis précédemment peuvent être préparés par réaction d'un composé de formule (IV) tel que défini ci-dessus, avec un composé de formule (V) tel que défini ci-dessus selon des méthodes connues de l'homme du métier telles que décrites dans WO03/104225. Plus particulièrement, le procédé de préparation des composés de formule générale (I) comprend la réaction d'un composé de formule (IV) : 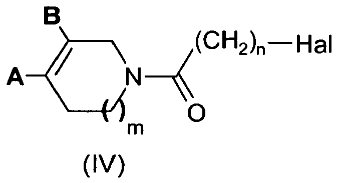 dans laquelle A, B, m et n sont définis comme en formule générale (I) et Hal représente un atome d'halogène, par exemple le chlore,
et d'un composé de formule générale (V) : 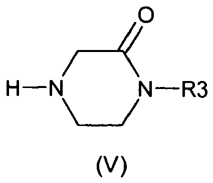 dans laquelle R3 est défini comme en formule générale (I) en présence d'une base, dans un solvant tel que décrit dans WO 03/104225. Ainsi, à titre de base, on peut citer les bases organiques telles que la triéthylamine, la N,N-diisopropylamine, la diisopropyl-éthylamine (DPEA), ou la N-méthyl-morpholine ou les carbonates ou bicarbonates de métal alcalin tel que le carbonate de potassium, le carbonate de sodium ou le bicarbonate de sodium et en l'absence ou en présence d'un iodure de métal alcalin tel que l'iodure de potassium ou l'iodure de sodium. La réaction s'effectue favorablement dans un solvant tel que l'acétonitrile, le N,N-diméthylformamide (DMF),I'N-méthyl-pyrropidone (NMP) le toluène ou le propan-2-ol, à une température comprise entre la température ambiante et la température de reflux du solvant. Par température ambiante, on entend une température comprise entre 5 et 25°C. A titre d'exemple, la réaction peut s'effectuer en présence de bicarbonate de sodium, d'iodure de sodium dans un solvant tel que le DMF. La réaction est préférentiellement conduite dans un réacteur à micro-ondes.

Dans les produits de formule générale (I) ainsi obtenus, R, R1, R2, R4, R5, R6, et R7 peuvent être modifiés par des traitements communément utilisés par l'homme du métier, comme par exemple par hydrolyse d'un groupe ester pour donner un groupe carboxylique.

Les sels d'addition à un acide des composés de formule générale (I) peuvent être obtenus par addition de l'acide approprié au composé de formule (I) sous la forme de base libre.

Les composés de formule (IV), dans laquelle R1 et R2 sont tels que définis pour les composés de formule (I), peuvent être préparés par réaction d'un composé de formule (II) avec un composé de formule (III) selon des méthodes connues de l'homme du métier par exemple en présence d'une base dans un solvant tel que décrit dans WO03/104225.

Plus particulièrement, les composés de formule (IV) peuvent être obtenus par réaction d'un composé correspondant de formule (II) : 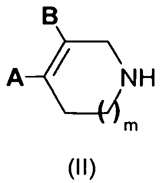 dans laquelle A, B et m sont définis comme en formule générale (I) ; éventuellement sous forme de sel d'addition à un acide, et d'un composé de formule (III) : 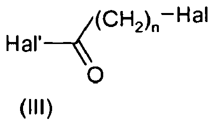 dans laquelle n et Hal sont tels que défini en formule (IV) et Hal' représente un atome d'halogène, identique ou différent. De préférence, Hal' représente un atome de chlore.

Cette réaction s'effectue généralement en présence d'une base, telle que la triéthylamine, la N,N-diisopropyléthylamine ou la N-méthylmorpholine, dans un solvant tel que le dichlorométhane, le chloroforme, le tétrahydrofurane, le dioxane ou un mélange de ces solvants et à une température comprise entre 0°C et la température ambiante. Les composés de formule (II) et (III) sont généralement disponibles commercialement ou peuvent être préparés selon des méthodes connues de l'homme du métier.

Les composés de formule (V), dans laquelle R3 est tel que défini dans la formule (I), sont préparés selon des méthodes connues de l'homme du métier. Ils peuvent être préparés, par exemple, selon le procédé qui suit :

### Schéma 2 :

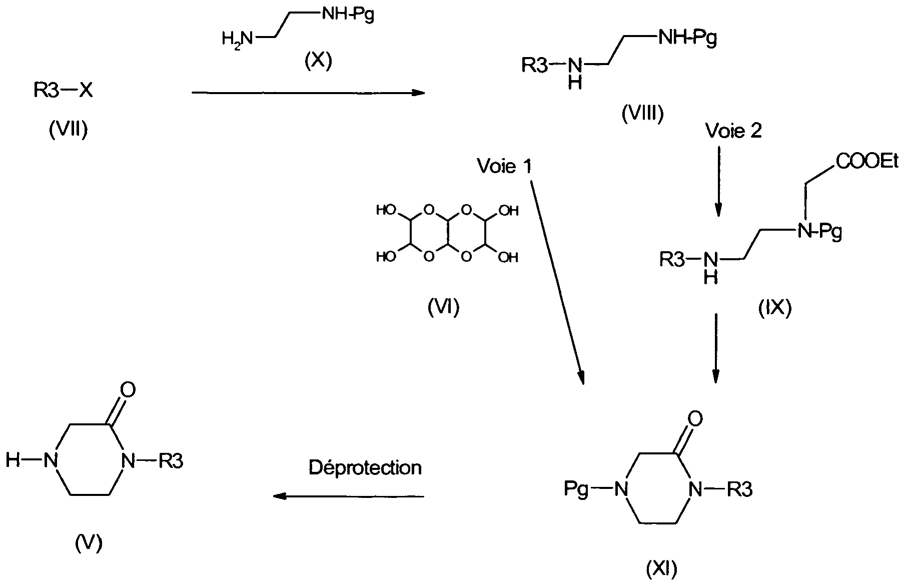

Plus particulièrement, le composé de formule (V) peut être préparé à partir d'un composé de formule (XI) correspondant, dans lequel R3 est tel que défini en formule (I) et Pg représente un groupe protecteur de l'atome d'azote, tel que le benzyle. Cette réaction peut s'effectuer par application ou adaptation de toute méthode connue de l'homme du métier ; généralement, cette réaction s'effectue en milieu acide, en présence d'un catalyseur, tel que Pd/C.

Le composé de formule (XI) peut être obtenu à partir d'un composé de formule (VIII) dans laquelle R3 est tel que défini en formule (I) et Pg représente un groupe protecteur, en présence d'un composé de formule (VI). Généralement, cette réaction s'effectue à température comprise entre la température ambiante et la température d'ébullition du mélange réactionnel pouvant comprendre de l'eau.

De façon alternative, le composé de formule (XI) peut être obtenu à partir d'un composé de formule (IX) par réaction avec des hydrures alkalines dans des solvants inertes tels que le toluène, le diméthylformamide ou diméthylsulphoxide à température comprise entre la température ambiante et la température d'ébullition du mélange réactionnel.

Le composé de formule (IX) peut être obtenu à partir d'un composé de formule (VIII) par réaction avec du bromo ou chloro acétate d'éthyl dans des solvants tels que le butanol ou l'acétone en présence d'une base tel que le carbonate de potassium à température comprise entre la température ambiante et la température d'ébullition du mélange réactionnel.

Le composé de formule (VIII) peut être obtenu à partir d'un composé de formule (VII) dans laquelle R3 est tel que défini en formule (I) et X représente un atome d'halogène, tel que le chlore, en présence d'un composé de formule (X) dans laquelle Pg représente un groupe protecteur tel que défini en formule (VIII). Généralement, cette réaction est conduite à température comprise entre la température ambiante et la température d'ébullition du mélange réactionnel.

Eventuellement, le procédé selon l'invention comprend l'étape ultérieure consistant à isoler le produit désiré obtenu.

Selon un autre de ses aspects, l'invention a également pour objet des composés de formule (IV) 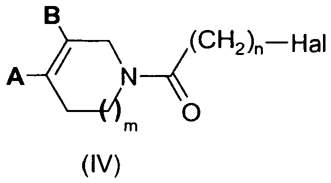 dans laquelle A, B, m et n sont définis comme en formule générale (I) et Hal représente un atome d'halogène, par exemple le chlore ; à l'état de bases ou de sels d'addition à des acides.

Ces composés sont utiles comme intermédiaires de synthèse des composés de formule (I).

Les composés de départ et les réactifs, dans les schémas 1 et 2, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

L'HPLC a été effectuée au moyen d'un système ThermoElectron LCQ Deca XP Max équipé avec un détecteur à spectrométrie de masse à trappe d'ions ainsi qu'un détecteur à barre de diodes.

Les conditions d'analyse par chromatographie liquide couplée à la spectrométrie de masse (LC/UV/MS) sont les suivantes:

### - système chromatographique A

- Eluant A = H₂O + 0,01 % TFA
- Eluant B = CH₃CN
- Gradient de 98% de A à 95% de B en 10 minutes, puis élution par 95% de B pendant 5 minutes.
- Débit 0,5 ml/minute; température 40°C
- Injection de 2 µL de solution à 0,1mg/ml dans un mélange CH₃CN : H₂O = 9 :1

### - système chromatographique B

- Eluant A = H₂O + 0,05% TFA
- Eluant B = CH₃CN + 0,035% TFA
- Gradient de 98% de A à 95% de B en 12 minutes, puis élution par 95% de B pendant 3 minutes.
- Débit 0,7 ml/minute; température 40°C
- Injection de 2 µL de solution à 0,1mg/ml dans un mélange CH₃CN : H₂O = 9 :1

### - système chromatographique C

- Eluant A = Tampon acétate d'ammonium 5mM pH 6.5
- Eluant B = CH₃CN
- Gradient de 98% de A à 95% de B en 10 minutes, puis élution par 95% de B pendant 5 minutes.
- Débit 0,5 ml/minute; température 40°C
- Injection de 2 µL de solution à 0,1mg/ml dans un mélange CH₃CN : H₂O = 9 :1 Les produits sont détectés en UV à 220 nm.

Les colonnes utilisées sont des C18 avec une granulométrie entre 2 et 4 µm, de préférence de 3,5µm.

Pour la partie spectrométrie de masse :
- Mode d'ionisation: électrospray positif (API-ES polarité+)
- Balayage de 120 à 1500 uma

Les spectres de résonnance magnétique nucléaire du proton (RMN ¹H) ont été enregistrés dans les conditions suivantes:
a) à 500 MHz sur un appareil Bruker équipé avec une console Avance III ;
b) à 400MHz sur un appareil Bruker équipé avec une console Avance I.

Les déplacements chimiques sont rapportés en ppm par rapport à la fréquence TMS.

Touts les spectres ont étés enregistrés à la température de 40°C.

Les abréviations utilisées pour caractériser les signaux sont les suivantes: s = singulet, bs =singulet large, m = multiplet, bm= multiplet large, d = doublet, bd= doublet large, t = triplet, q = quadruplet.
* = non intégrable à cause de l'interférence avec un pic large du à l'eau.
** = non intégrable à cause de l'interférence avec un pic du au solvant RMN. 2Xm= deux multiplet partiellement superposés.

### Préparation 1

### Chlorhydrate du 1-(5-trifluorométhyl-pyridin-2-yl)-pipérazine-2-one

On chauffe à 135° C pendant 6 heures dans un ballon, 10 g de 2-chloro-5-(trifluorométhyl)-pyridine (composé de formule (VII)) et 40,5 ml de N-benzyléthylèndiamine (composé de formule (X)). On verse dans l'eau et on extrait avec de l'acétate d'éthyle. On sèche et évapore sous vide ; le brut ainsi obtenu est purifié par flash chromatographie. Le produit isolé (composé de formule (VIII)), 14 g, est solubilisé dans 200 ml d'une solution 2N de HCl. On ajoute 30 g de dihydrate de glyoxal trimérique (composé VI), et laisse agiter à température ambiante pendant 72 heures. On extrait avec de l'acétate d'éthyle. On sèche et évapore sous vide; le brut ainsi obtenu est purifié par flash chromatographie. Le produit isolé (composé de formule (XI)), 10 g, est solubilisé dans 450 ml d'éthanol, puis on ajoute 15 ml d'une solution d'isopropanol saturé avec HCl et 3 g de Pd/C à 10%. On laisse à réagir sous flux d'hydrogène pendant 4 heures à la température de 40°C. On filtre et on évapore sous vide et on obtient 3 g du composé du titre. (composé de formule (V)) Pf 205-207°C.

### Préparation 2

### Chlorhydrate du 1-(5-méthyl-pyridin-2-yl)-pipérazine-2-one

On chauffe à 135° C pendant 5 heures dans un ballon, 4,7 g de 2-chloro-5-méthyl-pyridine (composé de formule (VII)) et 27,5 ml de N-benzyléthylèndiamine (composé de formule (X)). On verse dans l'eau et on extrait avec de l'acétate d'éthyle. On sèche et évapore sous vide ; le brut ainsi obtenu est purifié par flash chromatographie. Un produit de 3,6 g est isolé (composé de formule (VIII).

1,5 g de ce produit sont solubilisés dans 3 ml de butanol. On ajoute 0,85 g de carbonate de potassium et 1,05 g de bromoacétate d'éthyle et on chauffe à la température de reflux pendant 3 heures. On verse dans l'eau et on extrait avec de l'acétate d'éthyle. On sèche et évapore sous vide; le brut ainsi obtenu (2 g) est purifié par flash chromatographie. Le produit isolé (composé de formule (IX)), 1 g est ensuite solubilisé dans du toluène puis cette solution est ajoutée lentement sous flux d'azote à une suspension de 0,25 g de sodium hydrure 60% (NaH) dans 25 ml de toluène. On chauffe à la température de reflux pendant 2 heures. On verse dans l'eau et on extrait avec de l'acétate d'éthyle. On sèche et évapore sous vide.

On obtient 0,6 g de brut sous forme d'huile (composé de formule (IX)) que l'on solubilise dans 25 ml d'éthanol, puis on ajoute 1,5 ml d'une solution d'isopropanol saturé avec HCl et 0,3 g de Pd/C à 10%. On laisse à réagir sous flux d'hydrogène pendant 4 heures à la température de 40°C. On filtre et on évapore sous vide et on obtient 0,3 g du composé du titre. (composé de formule (V))

### Préparation 3

### 2-Chloro-1-[4-[3-trifluorométhyl-4-chloro-phényl]-1-[3,6-dihydro-1(2H)-pyridinyl]]-1-éthanone

On refroidit à 0°C du Chlorhydrate de 4-[3-(trifluorométhyl)-4-chloro-phényl]-3,6-dihydro-1(2*H*)-pyridine (composé de formule (II)) (3,94g) et 3,8 ml de triéthylamine dans 33,5 ml de dichlorométhane. On ajoute goutte à goutte du chlorure de 2-chloroacétyle (composé de formule (III)) et on laisse 1h30 sous agitation. On ajoute de l'eau et extrait au dichlorométhane. La phase organique est séchée sur Na₂SO₄, filtrée et évaporée sous vide.

On obtient 4,2 g du composé sous forme solide amorphe. (composé de formule (IV)).

### Exemple 1 :

### Composé n°1 : 4-{2-[4-(4-chloro-3-trifluorométhyl-phényl)-3,6-dihydro-2H-pyridin-1-yl]-2-oxo-éthyl}-1-(5-trifluorométhyl-pyridin-2-yl)-pipérazin-2-one et son chlorhydrate

On chauffe dans un réacteur à micro-ondes à 180°C pendant 30 minutes, un mélange de composé de la préparation 3 (0,49 mg), de composé de la préparation 1 (0,4 mg), de carbonate de potassium (0,41 g), d'iodure de sodium (0,45g) dans 7 ml de diméthylformamide. Le mélange réactionnel est versé dans l'eau et extrait à l'acétate d'éthyle. La phase organique est séchée sur Na₂SO₄, filtrée et évaporée pour donner 700 mg de brut sous forme d'huile. On purifie par chromatographie sur colonne de gel de silice en éluant par un mélange cyclohexane/acétate d'éthyle = 1/1. On prépare le chlorhydrate par addition d'une solution d'acide chlorhydrique dans l'isopropanol. On obtient 200 mg du composé du titre.

M+H⁺ = m/z 547

δ (ppm, dmso-d6): 2,55 (bs, **); 2,63 (m, **); 3,43-3,54 (m, 2H); 3,65 (m, *); 3,75 (m,1H); 3,96 (bs, 2H); 4,18 (m, 6H); 6,40 (bs, 1H); 7,72 (d, J = 8,4Hz, 1H), 7,74-7,80 (m, 1 H), 7,80-7,85 (m, 1H), 8,21 (d, J = 9Hz, 1 H); 8,26 (dd, J₁ = 9Hz, J₂ = 2Hz, 1 H); 8,88 (s, 1 H).

### Exemple 2 :

### Composé n°2 : 4-{2-[4-(4-chloro-3-trifluorométhyl-phényl)-3,6-dihydro-2H-pyridin-1-yl]-2-oxo-éthyl}-1-(5-méthyl-pyridin-2-yl)-pipérazin-2-one et son chlohydrate

En opérant comme décrit dans l'exemple 1, mais en utilisant le composé de la préparation 2 au lieu du composé de la préparation 1 on obtient le composé du titre.

M+H⁺ = m/z 493

(Appareil a). δ (ppm, dmso-d6): 2,32 (s, 3H), 2,56 (m, 1H), 2,65 (m, 1H), 3,10 - 3,60 (m, *), 3,64 (m, 1H), 3,77 (m, 1H), 3,98 (m, 2H), 4,05 - 4,51 (m, 6H), 6,41 (m, 1H), 7,69 (dd, J= 8,4 and 2.0Hz, 1H), 7,73 (d, 8.5Hz, 1H), 7,75 - 7,81 (m, 2H), 7,83 (bd, J= 9,3Hz, 1H), 8,31 (bd, J= 2Hz, 1H).

Le tableau qui suit illustre les structures chimiques et les propriétés physiques de quelques exemples de composés selon l'invention. Dans ce tableau :
- dans la colonne « sel », « - » représente un composé sous forme de base libre, alors que « HCl » représente un composé sous forme de chlorhydrate.

**Tableau**

| | | | | | | | | |
|---|---|---|---|---|---|---|---|---|
| 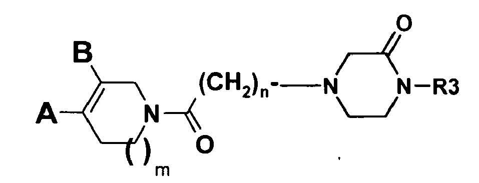 | | | | | | | | |

| **N** | **A** | **B** | **m** | **R3** | **n** | **Sel** | **PF** | **LCMS** |
|---|---|---|---|---|---|---|---|---|
| **1** | 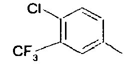 | H | 1 | 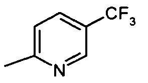 | 1 | HCl | 210-211 | MH+ 547 r.t.7,6 Méthode A |
| **2** | 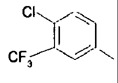 | H | 1 | 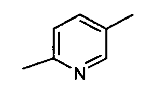 | 1 | HCl | 236-239 | MH+ 493 r.t. 6,5 Méthode A |
| **3** | 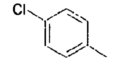 | H | 1 | 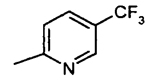 | 1 | - | 141-143 | MH+ 479 r.t. 7,9 Méthode C |
| **4** | 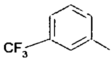 | H | 1 | 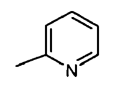 | 1 | - | 104-106 | MH+ 445 r.t. 5,4 Méthode A |
| **5** | 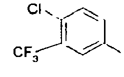 | H | 1 | 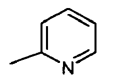 | 1 | HCl | 160-163 | - |
| **6** | 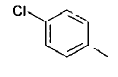 | H | 1 | 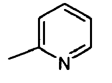 | 1 | - | 112-115 | MH+ 411 r.t. 5,4 Méthode A |
| **7** | 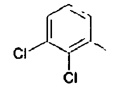 | H | 1 | 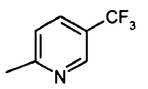 | 1 | - | 145-146 | MH+ 513 r.t. 6,9 Méthode A |
| **8** | 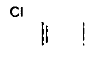 | H | 1 | 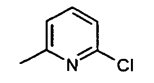 | 1 | HCl | 180-181 | MH+ 445 r.t. 7,2 Méthode A |
| **9** | 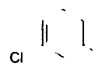 | H | 1 | 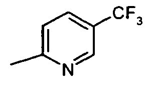 | 1 | HCl | 147-151 | MH+ 479 r.t. 7,3 Méthode A |
| **10** | 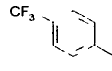 | H | 1 | 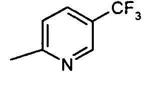 | 1 | - | 150-152 | MH+ 513 r.t. 7,9 Méthode C |
| **11** | 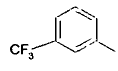 | H | 1 | 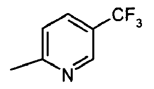 | 1 | Oxalate | 161-163 | MH+ 513 r.t. 6,8 Méthode A |
| **12** | 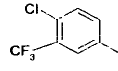 | H | 1 | 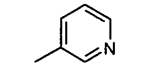 | 1 | - | - | M+ = 479 r.t. 5,7 Méthode A |
| **13** | 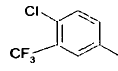 | H | 1 | 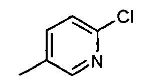 | 1 | fumarat e | - | M+ = 513 r.t. 6,5 Méthode A |
| **14** | H | 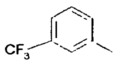 | 1 | 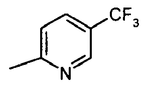 | 1 | Oxalate | 172-173 | M+ = 513 r.t.6,9 Méthode B |
| **15** | 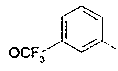 | H | 1 | 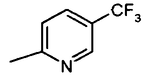 | 1 | Oxalate | 165-166 | M+ = 529 r.t. 7,1 Méthode A |
| **16** | 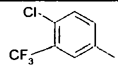 | H | 0 | 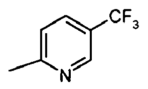 | 1 | - | 210-211 | M+ = 533 r.t. 7.0 Méthode A |
| **17** | 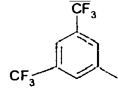 | H | 1 | 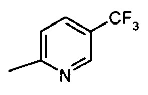 | 1 | Oxalate | 160-161 | M+ =581 r.t.8.1 Méthode C |
| **18** | 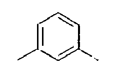 | H | 1 | 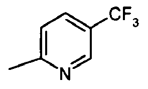 | 1 | - | 130-131 | M+ =459 r.t.7.2 Méthode C |
| **19** | 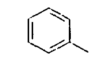 | H | 1 | 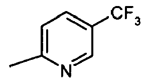 | 1 | - | 140-142 | M+ =445 r.t.6.0 Méthode A |
| **20** | H | 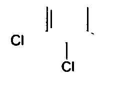 | 1 | 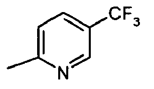 | 1 | Oxalate | 177-178 | M+ = 513 r.t. 6.6 Méthode A |
| **21** | H | 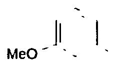 | 1 | 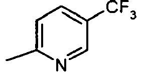 | 1 | Oxalate | 194-195 | M+ = 475 r.t. 5.9 Méthode A |

Les composés selon l'invention ont fait l'objet d'études biochimiques.

Culture cellulaire :
La souche SH-SY-5Y (neuroblastome humain) est cultivée classiquement dans un milieu de culture DMEM (de l'anglais Dulbecco's Modified Eagle's Medium) (Gibco BRL, France) contenant du SVF (5%) (sérum de veau foetal) (Boehringer Mannheim, Allemagne), du pyruvate de sodium (1 mM) et de la glutamine (4 mM) dans des flacons de culture recouvert de collagène (Becton Dickinson, France).
La souche mère SK-N-BE (neuroblastome humain) et le clone Bep 75, exprimant de manière stable la forme entière du récepteur p75^{NTR} humain (SK-N-BE Bep 75) sont cultivés classiquement dans un milieu de culture RPMI contenant du SVF (5%), du pyruvate de sodium (1 mM) et de la glutamine (4 mM). Pour les cellules SK-N-BE Bep 75, on ajoute de l'hygromycine (200 µl/20ml milieu) comme agent de sélection.

Etude de la liaison du ¹²⁵ I NGF au récepteur p75^{NTR}

L'étude de la liaison du NGF (le facteur de croissance neuronale radio- marqué à l'iode-125, Amersham - 2000 Ci/mmol) est réalisée sur une suspension cellulaire de la souche SK-N-BE Bep 75 en accord avec la méthode décrite par Weskamp (Neuron, 1991,6, 649-663). La liaison non spécifique est déterminée par la mesure de la liaison totale après une heure de pré-incubation avec les cellules à 37°C en présence de NGF non-radio marqué (1 µM). La liaison spécifique est calculée par différence entre la mesure de la liaison totale et la mesure de la liaison non-spécifique. Les expériences de compétition sont réalisées en utilisant une concentration en NGF-iodé (¹²⁵I NGF) de 0,3 nM. Les concentrations inhibitrices de 50 %(Cl₅₀) de la fixation de ¹²⁵I NGF au récepteur p75^{NTR} des composés selon l'invention sont faibles et varient de 10⁻⁶ à 10⁻¹¹M.

Les composés de formule (I) présentent une activité dans ce test avec des Cl₅₀ qui varient de 10⁻⁶ à 10⁻¹¹M.

Par exemple, les composés des exemples n° 3 et 1 ont montré respectivement une Cl₅₀ de 0,1 nM et 5,2 nM.

Etude de la dimérisation du récepteur p75^{NTR} indépendamment de son ligand

L'étude de la dimérisation du récepteur p75^{NTR} est réalisée sur une suspension cellulaire de la souche SK-N-BE Bep 75. Les cellules (2,5 10⁴ cellules/puits) sont disposées dans des puits (plaque de 96 puits) pendant 24 h, puis pré-incubées pendant 1 h à 37°C en présence ou non des composés selon l'invention. On ajoute ensuite du surnageant, issu de culture de cellules humaines d'origine rénale HEK293 exprimant, après 48h de transfection, et sécrétant une forme soluble du récepteur p75^{NTR} (partie extracellulaire du récepteur) couplée à une phosphatase alcaline, ce à la concentration finale de 10 nM. La quantification de la liaison spécifique du récepteur soluble p75^{NTR} au récepteur présent sur cellules SK-N-BE Bep 75 est déterminée par la mesure de l'activité enzymatique de la phosphatase alcaline après incubation des cellules pendant 1 heure à 37°C en présence du surnageant. Après filtration et transfert des filtres dans des plaques de 24 puits, l'activité de la phosphatase alcaline est déterminée par ajout de substrat chimioluminescent CDP-Star (ready-to-use, Roche). Les concentrations inhibitrices de 50 %(Cl₅₀) de la dimérisation du récepteur p75^{NTR} des composés selon l'invention sont faibles et varient de10⁻⁶ à 10⁻¹¹M.

Par exemple, les composé des exemples n°1, 2 et 3 ont montré respectivement des Cl₅₀ de 1,34 nM , 3,88 nM et 0,11 nM.

### Mesure de l'apoptose

Les cellules (souches de neuroblastomes humains SH-SY-5Y et SK-N-BE Bep 75) sont installées dans des boîtes de Pétri de 35 mm de diamètre (Biocoat collagenl, (10⁵ cellules/puits) dans un milieu de culture approprié contenant 5 % de SVF durant 24H. Le milieu de culture est ensuite éliminé, les cellules sont rincées avec du PBS (de l'anglais Dulbecco's Phosphate buffered saline), puis on ajoute soit du milieu frais contenant 5% de SVF, soit du milieu contenant du NGF (à la concentration de 10 ng/ml), soit du peptide beta-amyloïde (Aβ1-40) (à la concentration de 10 µM), ceci en présence ou non des composés selon l'invention. Les taux d'apoptose sont mesurés 48 heures après les traitements dans le cas de la souche SH-SY-5Y, et 24 heures après dans le cas de la souche SK-N-BE Bep 75 par quantification des histones cytoplasmiques associés aux fragments d'ADN (cell death détection ELISA, Boehringer Mannheim, Allemagne). Les taux d'apoptose sont exprimés en quantité d'oligonucléosomes/10⁵ cellules. Chaque valeur correspond à la moyenne de 9 points expérimentaux répartis dans 3 expériences indépendantes.

Les composés de formule (I) présentent une activité inhibitrice de l'apoptose induite par le NGF avec des Cl₅₀ qui varient de 10⁻⁶ à 10⁻¹¹M. Par exemple, le composé de l'exemple n°1 a montré une Cl₅₀ de 1,61 nM et le composé de l'exemple n°5 a montré une Cl₅₀ de 52 nM.

Ainsi, la fixation des composés selon l'invention au récepteur p75^{NTR} se traduit, d'une part, au niveau biochimique par l'inhibition de la dimérisation du récepteur induit par les neurotrophines, ou indépendamment du ligand, et, d'autre part, au niveau cellulaire par l'inhibition de l'effet proapoptotique médié par le récepteur p75^{NTR}.

Ainsi, selon un des objets de la présente invention, les composés de formule (I) présentent une activité très intéressante d'inhibition de la dimérisation du récepteur p75^{NTR} indépendamment de son ligand.

Les composés selon l'invention peuvent donc être utilisés pour la préparation de médicaments, en particulier de médicaments destinés à prévenir ou à traiter toute pathologie où le récepteur p75^{NTR} est impliqué.

Ainsi, selon un autre de ses aspects, l'invention a pour objet des médicaments qui comprennent un composé de formule(I), ou un sel d'addition de ce dernier à un acide pharmaceutiquement acceptable.

Selon un autre de ses aspects, l'invention a pour objet un composé de formule(I), ou un sel d'addition de ce dernier à un acide pharmaceutiquement acceptable pour la prévention ou le traitement des pathologies ci-après indiquées.

Ainsi, les composés selon l'invention peuvent être utilisés, chez l'homme ou chez l'animal, dans le traitement ou la prévention de différentes affections p75^{NTR} dépendantes telles que les maladies neurodégénératives centrales et périphériques comme la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire ; la sclérose latérale amyotrophique, la sclérose en plaques ; les affections cardiovasculaires comme les dommages cardiaques post-ischémiques, les cardiomyopathies, l'infarctus du myocarde, l'insuffisance cardiaque, l'ischémie cardiaque, l'infarctus cérébral ; les neuropathies périphériques (d'origine diabétique, traumatisme ou iatrogène) ; les dommages au nerf optique et à la rétine (dégénérescence du pigment rétinien, glaucome) ; l'ischémie rétinale ; la dégénérescence maculaire ; les traumatismes de la moelle épinière et les traumatismes crâniens ; l'athérosclérose ; les sténoses ; les désordres de la cicatrisation ; l'alopécie.

Les composés selon l'invention peuvent également être utilisés dans le traitement des cancers comme celui du poumon, de la thyroïde, du pancréas, de la prostate, de l'intestin grêle et du colon, du sein, dans le traitement des tumeurs, des métastases et des leucémies.

Les composés selon l'invention peuvent également être utilisés dans le traitement des désordres respiratoires comme l'inflammation pulmonaire, l'allergie et l'asthme, la broncho-pneumopathie chronique obstructive.

Les composés selon l'invention peuvent aussi être utilisés dans le traitement de la douleur cutanée (de la peau, des tissus sous-cutanés et organes associés), somatique, viscérale (au niveau du système circulatoire, respiratoire, gastro-intestinal, ou génito-urinaire), et neurologiques.

Les composés selon l'invention peuvent être utilisés dans le traitement des douleurs chroniques neuropathiques et inflammatoires et dans le traitement des maladies auto-immunes comme la polyarthrite rhumatoïde.

Les composés selon l'invention peuvent aussi être utilisés dans le traitement des maladies comme la spondylarthrite ankylosante, le rhumatisme psoriasique, le psoriasis en plaques.

Les composés selon l'invention peuvent également être utilisés dans le traitement des fractures osseuses, dans le traitement ou la prévention des maladies osseuses comme l'ostéoporose.

Ainsi la présente invention a pour objet un composé de formule (I) selon l'invention pour prévenir ou à traiter toute pathologie où le récepteur p75^{NTR} est impliqué ou plus particulièrement les pathologies tels qu'indiquées ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel peut être administré sous forme unitaire d'administration, en mélange avec des excipients pharmaceutiques classiques, aux animaux et aux êtres humains pour la prévention ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La dose de principe actif administrée par jour peut atteindre 0,01 à 100 mg/kg, en une ou plusieurs prises, préférentiellement 0,02 à 50 mg/kg. En général, la dose journalière du composé de l'invention sera la dose efficace la plus faible du composé capable de produire un effet thérapeutique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé de formule (I) : 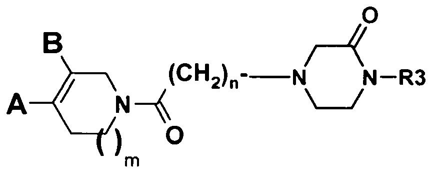 dans laquelle :
- m représente 0 ou 1 ;
- A représente : 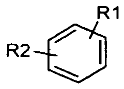 et B représente un atome d'hydrogène
ou
A représente un atome d'hydrogène et B représente : 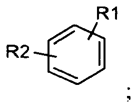
- R1 et R2, identiques ou différents, représentent indépendamment un atome d'hydrogène ou d'halogène, un groupe C1-4 alkyle, C1-4 fluoroalkyle, C1-2 perfluoroalkyle, C1-4 alcoxy ou un groupe trifluorométhoxy;
- n est 1 ou 2 ;
- R3 représente un groupe de formule: 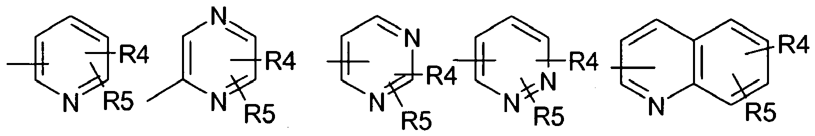 où R4 et R5, identiques ou différents, sont situés sur des positions quelconques disponibles, et représentent indépendamment un atome d'hydrogène ou d'halogène, un hydroxyle, un groupe C1-4 alkyle, C1-4 fluoroalkyle, C1-2 perfluoroalkyle, C1-4 alcoxy, un groupe trifluorométhoxy, un groupe cyano, un groupe COOH, COOalkyle, CONH2, CONR6R7 ou NHCOR.
- R,R6 et R7 représentent un C1-C6 alkyle;
à l'état de bases ou de sels d'addition à des acides.

2. Composé selon la revendication 1 tel que R 4 et R5, identiques ou différents, sont situés sur des positions quelconques disponibles, et représentent indépendamment CONH2, CONR6R7 ou NHCOR ; à l'état de bases ou de sels d'addition à des acides.

3. Composé selon la revendication 1 tel que :
- m représente 1 ;
- A représente : 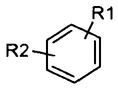 et B représente un atome d'hydrogène ;
- R1 et R2, identiques ou différents, représentent indépendamment un atome d'hydrogène ou d'halogène, un groupe C1-4 alkyle, C1-4 fluoroalkyle, C1-2 perfluoroalkyle, C1-4 alcoxy ou un groupe trifluorométhoxy;
- n est 1 ou 2 ;
- R3 représente un groupe de formule: 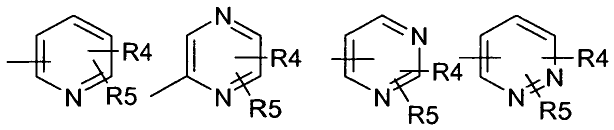 où R 4 et R5, identiques ou différents, sont situés sur des positions quelconques disponibles, et représentent indépendamment un atome d'hydrogène ou d'halogène, un hydroxyle, un groupe C1-4 alkyle, C1-4 fluoroalkyle, C1-2 perfluoroalkyle, C1-4 alcoxy, un groupe trifluorométhoxy, un groupe cyano, un groupe COOH ou COOalkyle ;
à l'état de bases ou de sels d'addition à des acides.

4. Composé selon l'une quelconque des revendications précédentes tel que R1 est différent de H ; à l'état de bases ou de sels d'addition à des acides.

5. Composé selon l'une quelconque des revendications précédentes tel que R1 est en position -2-, -3- ou -4- et représente un atome de chlore, un radical CF3 et R2 représente un hydrogène ou un 3- ou 4-CI ; à l'état de bases ou de sels d'addition à des acides.

6. Composé selon l'une quelconque des revendications précédentes tel que R3 représente un 2-pyridynyle ou un 2 -pyrimidinyle, chacun substitué par R4 et R5 tels que définis en revendication 1 ; à l'état de bases ou de sels d'addition à des acides.

7. Composé selon l'une quelconque des revendications précédentes, tel que n = 1 ; à l'état de bases ou de sels d'addition à des acides.

8. Composé selon l'une quelconque des revendications 1 et 3 à 7 tel que R1 représente le 3-CF3 ;
R2 représente le 4-Chloro ;
R3 représente un résidu 2-pyridyl substitué en 5 par un CF3 ; et
n=1;
à l'état de base ou de sels d'addition à des acides.

9. Composé selon l'une quelconque des revendications précédentes chois parmi :
Composé n°1 :4-{2-[4-(4-chloro-3-trifluorométhyl-phényl)-3,6-dihydro-2*H-*pyridin-1-yl]-2-oxo-éthyl}-1-(5-trifluorométhyl-pyridin-2-yl)-pipérazin-2-one ;
Composé n°2 : 4-{2-[4-(4-chloro-3-trifluorométhyl-phényl)-3,6-dihydro-2*H-*pyridin-1-yl]-2-oxo-éthyl}-1-(5-méthyl-pyridin-2-yl)-pipérazin-2-one ;
Composé n°3 : 4-{2-[4-(4-chloro-phényl)-3,6-dihydro-2*H*-pyridin-1-yl]-2-oxo-éthyl}-1-(5-trifiuorométhyl-pyridin-2-yl)-pipérazin-2-one ;
Composé n°4 : 4-{2-0xo-2-[4-(3-trifluorométhyl-phényl)-3,6-dihydro-2H-pyridin-1-yl]-éthyl}-1-pyridin-2-yl-pipérazin-2-one;
Composé n°5 : 4-{2-[4-(4-chloro-3-trifluorométhyl-phényl)-3,6-dihydro-2*H-*pyridin-1-yl]-2-oxo-éthyl}-1-pyridin-2-yl-pipérazin-2-one ;
Composé n°6 : 4-{2-[4-(4-chloro-phényl)-3,6-dihydro-2*H*-pyridin-1-yl]-2-oxo-éthyl}-1-pyridin-2-yl-pipérazin-2-one ;
Composé n°7 : 4-{2-[4-(2,3-dichloro-phényl)-3,6-dihydro-2*H*-pyridin-1-yl]-2-oxo-éthyl}-1-(5-trifluorométhyl-pyridin-2-yl)-pipérazin-2-one ;
Composé n°8 : 4-{2-[4-(4-chloro-phényl)-3,6-dihydro-2*H*-pyridin-1-yl]-2-oxo-éthyl}-1-(6-chloro-pyridin-2-yl)-pipérazin-2-one ;
Composé n°9 : 4-{2-[4-(3-chloro-phényl)-3,6-dihydro-2*H*-pyridin-1-yl]-2-oxo-éthyl}-1-(5-trifluorométhyl-pyridin-2-yl)-pipérazin-2-one ;
Composé n°10 : 4-{2-[4-(4-trifluorométhyl-phényl)-3,6-dihydro-2*H*-pyridin-1-yl]-2-oxo-éthyl}-1-(5-trifluorométhyl -pyridin-2-yl)-pipérazin-2-one ;
Composé n°11 : 4-{2-[4-(3-trifluorométhyl-phényl)-3,6-dihydro-2*H*-pyridin-1-yl]-2-oxo-éthyl}-1-(5-trifluorométhyl-pyridin-2-yl)-pipérazin-2-one ;
Composé n°12 : 4-{2-[4-(4-chloro-3-trifluorométhyl-phényl)-3,6-dihydro-2*H-*pyridin-1-yl]-2-oxo-éthyl}-1-pyridin-3-yl-pipérazin-2-one ;
Composé n°13: 1-(6-Chloro-pyridin-3-yl)-4-{2-[4-(4-chloro-3-trifluorométhyl-phényl)-3,6-dihydro-2H-pyridin-1-yl]-2-oxo-éthyl}-pipérazin-2-one;
Composé n°14 : 4-{2-Oxo-2-[5-(3-trifluorométhyl-phényl)-3,6-dihydro-2H-pyridin-1-yl]-éthyl}-1-(5-trifluorométhyl-pyridin-2-yl)-pipérazin-2-one;
Composé n°15 : 4-{2-Oxo-2-[4-(3-trifluorométhoxyl-phényl)-3,6-dihydro-2H-pyridin-1-yl]-éthyl}-1-pyridin-2-yl-pipérazin-2-one;
Composé n°16 : 4-{2-[4-(4-chloro-3-trifluorométhyl-phényl)-2,5-dihydro-pyrrol - 1-yl]-2-oxo-éthyl}-1-(5-trifluorométhyl-pyridin-2-yl)-pipérazin-2-one ;
- Composé n°17 : 4-{2-[4-(3,5bis-trifluorométhyl-phényl)-3,6-dihydro-2H-pyridin 1-yl]-2-oxo-éthyl}-1-(5-trifluorométhyl-pyridin-2-yl)-pipérazin-2-one ;
- Composé n°18 : 4-{2-[4-(3-méthyl-phényl)-3,6-dihydro-2H-pyridin -1-yl]-2-oxo-éthyl}-1-(5-trifluorométhyl-pyridin-2-yl)-pipérazin-2-one ;
- Composé n°19 : 4-{2-[4-phényl-3,6-dihydro-2H-pyridin -1-yl]-2-oxo-éthyl}-1-(5-trifluorométhyl-pyridin-2-yl)-pipérazin-2-one ;
- Composé n°20 : 4-{2-Oxo-2-[5-(2,3-dichloro-phényl)-3,6-dihydro-2H-pyridin-1-yl]-éthyl}-1-(5-trifluorométhyl-pyridin-2-yl)-pipérazin-2-one;
- Composé n°21 : 4-{2-Oxo-2-[5-(3-methoxy-phényl)-3,6-dihydro-2H-pyridin-1-yl]-éthyl}-1-(5-trifluorométhyl-pyridin-2-yl)-pipérazin-2-one;
à l'état de bases ou de sels d'addition à des acides.

10. Procédé de préparation d'un composé de formule (I) selon l'une quelconque des revendications précédentes dans laquelle A, B, m, n, R3 sont tels que définis précédemment comprenant l'étape de réaction d'un composé de formule (IV) 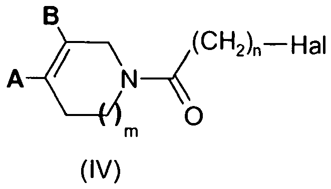 dans laquelle A, B, m, n sont définis comme en formule générale (I) et Hal représente un atome d'halogène,
et d'un composé de formule générale (V) : 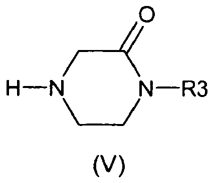 dans laquelle R3 est défini comme en formule générale (I), en présence d'une base.

11. Composé de formule (IV) 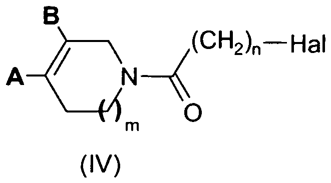 dans laquelle A, B, m et n sont définis selon l'une quelconque des revendications 1 à 9 et Hal représente un atome d'halogène ;
à l'exception du 2-Chloro-1-[4-(2-méthoxyphényly)-3,6-dihydro-2H-pyridin-1-yl]-éthanone et du 2-Chloro-1-[4-(4-bromophényl)-3,6-dihydro-2H-pyridin-1-yl]-éthanone ; à l'état de base ou de sels d'addition à des acides.

12. Médicament, **caractérisé en ce qu'**il comprend un composé de formule (I) selon l'une quelconque des revendications 1 à 9, ou un sel d'addition de ce composé à un acide pharmaceutiquement acceptable.

13. Composition pharmaceutique, **caractérisée en ce qu'**elle comprend un composé de formule (I) selon l'une quelconque des revendications 1 à 9, ou un sel pharmaceutiquement acceptable, ainsi qu'au moins un excipient pharmaceutiquement acceptable.

14. Composé de formule générale (I) selon l'une quelconque des revendications 1 à 9 pour la préparation d'un médicament destiné à la prévention ou le traitement de maladies neurodégénératives centrales et périphériques, la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire, la sclérose latérale amyotrophique, la sclérose en plaques, les affections cardiovasculaires, les dommages cardiaques post-ischémiques, les cardiomyopathies, l'infarctus du myocarde, l'insuffisance cardiaque, l'ischémie cardiaque, l'infarctus cérébral ; les neuropathies périphériques, les dommages au nerf optique et à la rétine, dégénérescence du pigment rétinien, glaucome, l'ischémie rétinale , la dégénérescence maculaire, les traumatismes de la moelle épinière, les traumatismes crâniens, l'athérosclérose, les sténoses, les désordres de la cicatrisation, l'alopécie, les cancers, les tumeurs, les métastases, les leucémies, les désordres respiratoires, l'inflammation pulmonaire, l'allergie, l'asthme, la broncho-pneumopathie chronique obstructive, la douleur cutanée, somatique, viscérale, et neurologique, les douleurs chroniques neuropathiques et inflammatoires, les maladies auto-immunes, la polyarthrite rhumatoïde, la spondylarthrite ankylosante, le rhumatisme psoriasique, le psoriasis en plaques, les fractures osseuses, les maladies osseuses, l'ostéoporose.

15. Composé selon la revendication 14 ayant la capacité d'inhiber la dimérisation du récepteur p75^{NTR} indépendamment de son ligand.

## Claims

1. Compound of formula (I): 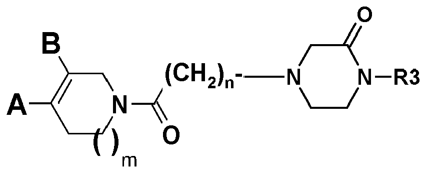 in which:
- m is 0 or 1;
- A is: 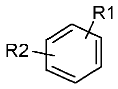 and B is a hydrogen atom or
A is a hydrogen atom and B is: 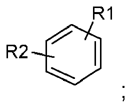
- R1 and R2, which may be identical or different, are independently a hydrogen or halogen atom, a C₁-C₄ alkyl, C₁-C₄ fluoroalkyl, C₁-C₂ perfluoroalkyl or C₁-C₄ alkoxy group or a trifluoromethoxy group;
- n is 1 or 2;
- R3 is a group of formula: 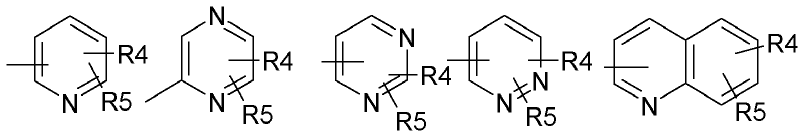 where R4 and R5, which may be identical or different, are located on any available positions, and are independently a hydrogen or halogen atom, a hydroxyl, a C₁-C₄ alkyl, C₁-C₄ fluoroalkyl, C₁-C₂ perfluoroalkyl, C₁-C₄ alkoxy group, a trifluoromethoxy group, a cyano group, a COOH, COOalkyl, CONH₂, CONR6R7 or NHCOR group,
- R, R6 and R7 are a C₁-C₆ alkyl;
in the form of bases or of addition salts with acids.

2. Compound according to Claim 1, such that R4 and R5, which may be identical or different, are located on any available positions, and are independently CONH₂, CONR6R7 or NHCOR; in the form of bases or of addition salts with acids.

3. Compound according to Claim 1, such that:
- m is 1;
- A is: 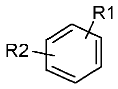 and B is a hydrogen atom;
- R1 and R2, which may be identical or different, are independently a hydrogen or halogen atom, a C₁-C₄ alkyl, C₁-C₄ fluoroalkyl, C₁-C₂ perfluoroalkyl or C₁-C₄ alkoxy group or a trifluoromethoxy group;
- n is 1 or 2;
- R3 is a group of formula: 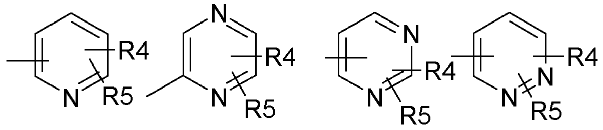 where R4 and R5, which may be identical or different, are located on any available positions and are independently a hydrogen or halogen atom, a hydroxyl, a C₁-C₄ alkyl, C₁-C₄ fluoroalkyl, C₁-C₂ perfluoroalkyl or C₁-C₄ alkoxy group, a trifluoromethoxy group, a cyano group, or a COOH or COOalkyl group;
in the form of bases or of addition salts with acids.

4. Compound according to any one of the preceding claims, such that R1 is other than H; in the form of bases or of addition salts with acids.

5. Compound according to any one of the preceding claims, such that R1 is in position -2-, -3- or -4- and is a chlorine atom, or a CF3 radical, and R2 is a hydrogen or a 3- or 4-Cl; in the form of bases or of addition salts with acids.

6. Compound according to any one of the preceding claims, such that R3 is a 2-pyridynyl or a 2-pyrimidinyl, each substituted with R4 and R5 as defined in Claim 1; in the form of bases or of addition salts with acids.

7. Compound according to any one of the preceding claims, such that n = 1; in the form of bases or of addition salts with acids.

8. Compound according to any one of the preceding Claims 1 and 3 to 7, such that
R1 is 3-CF₃;
R2 is 4-Chloro;
R3 is a 2-pyridyl residue 5-substituted with a CF₃; and
n = 1;
in the form of a base or of addition salts with acids.

9. Compound according to any one of the preceding claims chosen from:
Compound No.1: 4-{2-[4-(4-chloro-3-trifluoromethylphenyl)-3,6-dihydro-2*H*-pyridin-1-yl]-2-oxoethyl}-1-(5-trifluoromethylpyridin-2-yl)piperazin-2-one;
Compound No. 2: 4-{2-[4-(4-chloro-3-trifluoromethylphenyl)-3,6-dihydro-2*H*-pyridin-1-yl]-2-oxoethyl}-1-(5-methylpyridin-2-yl)piperazin-2-one;
Compound No. 3: 4-{2-[4-(4-chlorophenyl)-3,6-dihydro-2*H*-pyridin-1-yl]-2-oxoethyl}-1-(5-trifluoromethylpyridin-2-yl)piperazin-2-one;
Compound No. 4: 4-{2-oxo-2-[4-(3-trifluoromethylphenyl)-3,6-dihydro-2*H-*pyridin-1-yl]ethyl}-1-pyridin-2-ylpiperazin-2-one;
Compound No. 5: 4-{2-[4-(4-chloro-3-trifluoromethylphenyl)-3,6-dihydro-2*H*-pyridin-1-yl]-2-oxoethyl}-1-pyridin-2-ylpiperazin-2-one;
Compound No. 6: 4-{2-[4-(4-chlorophenyl)-3,6-dihydro-2*H*-pyridin-1-yl]-2-oxoethyl}-1-pyridin-2-yl-piperazin-2-one;
Compound No. 7: 4-{2-[4-(2,3-dichlorophenyl)-3,6-dihydro-2*H*-pyridin-1-yl]-2-oxoethyl}-1-(5-trifluoromethylpyridin-2-yl)piperazin-2-one;
Compound No. 8: 4-{2-[4-(4-chlorophenyl)-3,6-dihydro-2*H*-pyridin-1-yl]-2-oxoethyl}-1-(6-chloropyridin-2-yl)piperazin-2-one;
Compound No. 9: 4-{2-[4-(3-chlorophenyl)-3,6-dihydro-2*H*-pyridin-1-yl]-2-oxoethyl}-1-(5-trifluoromethylpyridin-2-yl)piperazin-2-one;
Compound No. 10: 4-{2-[4-(4-trifluoromethylphenyl)-3,6-dihydro-2*H-*pyridin-1-yl]-2-oxoethyl}-1-(5-trifluoromethylpyridin-2-yl)piperazin-2-one;
Compound No. 11: 4-{2-[4-(3-trifluoromethylphenyl)-3,6-dihydro-2*H-*pyridin-1-yl]-2-oxoethyl}-1-(5-trifluoromethylpyridin-2-yl)piperazin-2-one;
Compound No. 12: 4-{2-[4-(4-chloro-3-trifluoromethylphenyl)-3,6-dihydro-2*H*-pyridin-1-yl]-2-oxoethyl}-1-pyridin-3-yl-piperazin-2-one;
Compound No. 13: 1-(6-chloropyridin-3-yl)-4-{2-[4-(4-chloro-3-trifluoromethylphenyl)-3,6-dihydro-2H-pyridin-1-yl]-2-oxoethyl}piperazin-2-one;
Compound No. 14: 4-{2-oxo-2-[5-(3-trifluoromethylphenyl)-3,6-dihydro-2H-pyridin-1-yl]ethyl}-1-(5-trifluoromethylpyridin-2-yl)piperazin-2-one;
Compound No. 15: 4-{2-oxo-2-[4-(3-trifluoromethoxylphenyl)-3,6-dihydro-2H-pyridin-1-yl]ethyl}-1-pyridin-2-ylpiperazin-2-one;
Compound No. 16: 4-{2-[4-(4-chloro-3-trifluoromethylphenyl)-2,5-dihydropyrrol-1-yl]-2-oxoethyl}-1-(5-trifluoromethylpyridin-2-yl)piperazin-2-one;
- Compound No. 17: 4-{2-[4-(3,5-bistrifluoromethylphenyl)-3,6-dihydro-2H-pyridin-1-yl]-2-oxoethyl}-1-(5-trifluoromethylpyridin-2-yl)piperazin-2-one;
- Compound No. 18: 4-{2-[4-(3-methylphenyl)-3,6-dihydro-2H-pyridin-1-yl]-2-oxoethyl}-1-(5-trifluoromethylpyridin-2-yl)piperazin-2-one;
- Compound No. 19: 4-{2-[4-phenyl-3,6-dihydro-2H-pyridin-1-yl]-2-oxoethyl}-1-(5-trifluoromethylpyridin-2-yl)piperazin-2-one;
- Compound No. 20: 4-{2-oxo-2-[5-(2,3-dichlorophenyl)-3,6-dihydro-2H-pyridin-1-yl]ethyl}-1-(5-trifluoromethylpyridin-2-yl)piperazin-2-one;
- Compound No. 21: 4-{2-oxo-2-[5-(3-methoxyphenyl)-3,6-dihydro-2H-pyridin-1-yl]ethyl}-1-(5-trifluoromethylpyridin-2-yl)piperazin-2-one;
in the form of bases or of addition salts with acids.

10. Process for preparing a compound of formula (I) according to any one of the preceding claims, in which A, B, m, n, R3 are as defined above, comprising the step of reacting a compound of formula (IV) 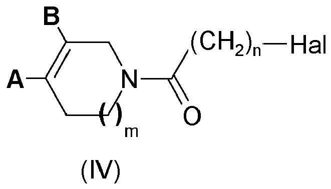 in which A, B, m and n are as defined in general formula (I) and Hal is a halogen atom,
and a compound of general formula (V): 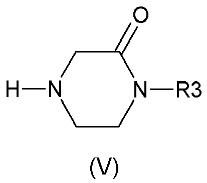 in which R3 is as defined in general formula (I), in the presence of a base.

11. Compound of formula (IV) 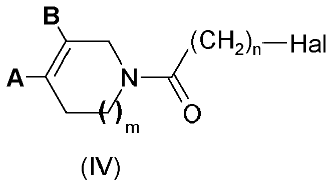 in which A, B, m and n are defined according to any one of Claims 1 to 9 and Hal is a halogen atom;
with the exception of 2-chloro-1-[4-(2-methoxyphenyl)-3,6-dihydro-2H-pyridin-1-yl]-ethanone and 2-chloro-1-[4-(4-bromophenyl)-3,6-dihydro-2H-pyridin-1-yl]ethanone;
in the form of a base or of addition salts with acids.

12. Medicament, **characterized in that** it comprises a compound of formula (I) according to any one of Claims 1 to 9, or an addition salt of this compound with a pharmaceutically acceptable acid.

13. Pharmaceutical composition, **characterized in that** it comprises a compound of formula (I) according to any one of Claims 1 to 9, or a pharmaceutically acceptable salt, and also at least one pharmaceutically acceptable excipient.

14. Compound of general formula (I) according to any one of Claims 1 to 9, for the preparation of a medicament for use in the prevention or treatment of central and peripheral neurodegenerative diseases, senile dementia, epilepsy, Alzheimer's disease, Parkinson's disease, Huntington's chorea, Down's syndrome, prion diseases, amnesia, schizophrenia, depression, bipolar disorder, amyotrophic lateral sclerosis, multiple sclerosis, cardiovascular conditions, post-ischaemic cardiac damage, cardiomyopathies, myocardial infarction, heart failure, cardiac ischaemia, cerebral infarction; peripheral neuropathies, damage to the optic nerve and to the retina, retinal pigment degeneration, glaucoma, retinal ischaemia, macular degeneration, spinal cord traumas, cranial traumas, atherosclerosis, stenoses, cicatrization disorders, alopecia, cancers, tumours, metastases, leukaemias, respiratory disorders, pulmonary inflammation, allergy, asthma, chronic obstructive pulmonary disease, cutaneous, somatic, visceral, and neurological pain, chronic neuropathic and inflammatory pain, autoimmune diseases, rheumatoid arthritis, ankylosing spondyl arthritis, psoriatic arthritis, plaque psoriasis, bone fractures, bone diseases and osteoporosis.

15. Compound according to Claim 14, which has the ability to inhibit p75^{NTR} receptor dimerization independently of its ligand.

## Patentansprüche

1. Verbindung der Formel (I): 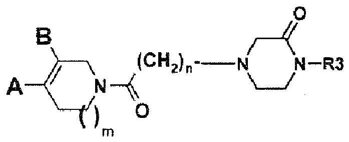 in der:
- m 0 oder 1 bedeutet; R1
- A 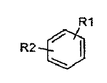 bedeutet;
und B ein Wasserstoffatom bedeutet
oder
A ein Wasserstoffatom bedeutet und B 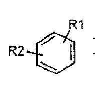 bedeutet;
- R1 und R2, die gleich oder verschieden sind, unabhängig voneinander ein Wasserstoff- oder Halogenatom, eine C1-4-Alkyl-, C1-4-Fluoralkyl-, C1-2-Perfluoralkyl-, C1-4-Alkoxy- oder Trifluormethoxygruppe bedeuten;
- n 1 oder 2 bedeutet;
R3 eine Gruppe der Formel: 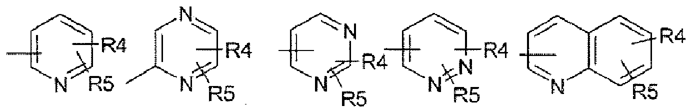 bedeutet, worin sich R4 und R5, die gleich oder verschieden sind, an beliebigen verfügbaren Positionen befinden und unabhängig voneinander ein Wasserstoff-oder Halogenatom, ein Hydroxyl, eine C1-4-Alkyl-, C1-4-Fluoralkyl-, C1-2-Perfluoralkyl-, C1-4-Alkoxy-, Trifluormethoxy-, Cyano-, COOH-, COO-Alkyl-, CONH2-, CONR6R7- oder NHCOR-Gruppe bedeuten,
- R, R6 und R7 C1-C6-Alkyl bedeuten;
als Basen oder als Säureadditionssalze.

2. Verbindung nach Anspruch 1, in der sich R4 und R5, die gleich oder verschieden sind, in beliebigen verfügbaren Positionen befinden und unabhängig voneinander CONH2, CONR6R7 oder NHCOR bedeuten; als Basen oder als Säureadditionssalze.

3. Verbindung nach Anspruch 1, in der:
- m 1 bedeutet;
- A 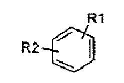 bedeutet
und B ein Wasserstoffatom bedeutet;
- R1 und R2, die gleich oder verschieden sind, unabhängig voneinander ein Wasserstoff- oder Halogenatom, eine C1-4-Alkyl-, C1-4-Fluoralkyl-, C1-2-Perfluoralkyl-, C1-4-Alkoxy- oder Trifluormethoxygruppe bedeuten;
- n 1 oder 2 bedeutet;
- R3 eine Gruppe der Formel 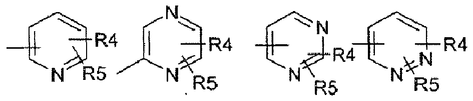 bedeutet, worin sich R4 und R5, die gleich oder verschieden sind, an beliebigen verfügbaren Positionen befinden und unabhängig voneinander ein Wasserstoff-oder Halogenatom, ein Hydroxyl, eine C1-4-Alkyl-, C1-4-Fluoralkyl-, C1-2-Perfluoralkyl-, C1-4-Alkoxy-, Trifluormethoxy-, Cyano-, COOH- oder COO-Alkyl-Gruppe als Basen oder als Säureadditionssalze.

4. Verbindung nach einem der vorhergehenden Ansprüche, in der R1 nicht H bedeutet; als Basen oder als Säureadditionssalze.

5. Verbindung nach einem der vorhergehenden Ansprüche, in der sich R1 in -2-, -3- oder -4-Position befindet und ein Chloratom, einen CF3-Rest bedeutet und R2 Wasserstoff oder 3- oder 4-Cl bedeutet; als Basen oder als Säureadditionssalze.

6. Zusammensetzung nach einem der vorhergehenden Ansprüche, in der R3 2-Pyridinyl oder 2-Pyrimidinyl bedeutet, die jeweils durch R4 und R5 wie in Anspruch 1 definiert substituiert sind; als Basen oder als Säureadditionssalze.

7. Verbindung nach einem der vorhergehenden Ansprüche, in der n = 1; als Basen oder als Säureadditionssalze.

8. Verbindung nach einem der Ansprüche 1 und 3 bis 7, in der
R1 3-CF3 bedeutet;
R2 4-Chlor bedeutet;
R3 einen 2-Pyridylrest, der in 5-Position durch CF3 substituiert ist, bedeutet; und
n = 1;
als Base oder als Säureadditionssalze.

9. Verbindung nach einem der vorhergehenden Ansprüche, ausgewählt aus der Reihe:
Verbindung Nr. 1: 4-{2-[4-(4-Chlor-3-trifluormethylphenyl)-3,6-dihydro-2*H*-pyridin-1-yl]-2-oxoethyl}-1-(5-trifluormethylpyridin-2-yl)piperazin-2-on;
Verbindung Nr. 2: 4-{2-[4-(4-Chlor-3-trifluormethylphenyl)-3,6-dihydro-2*H*-pyridin-1-yl]-2-oxoethyl}-1-(5-methylpyridin-2-yl)piperazin-2-on;
Verbindung Nr. 3: 4-{2-[4-(4-Chlorphenyl)-3,6-dihydro-2*H*-pyridin-1-yl]-2-oxoethyl}-1-(5-trifluormethylpyridin-2-yl)piperazin-2-on;
Verbindung Nr. 4: 4-{2-Oxo-2-[4-(3-trifluormethylphenyl)-3,6-dihydro-2H-pyridin-1-yl]ethyl}-1-pyridin-2-ylpiperazin-2-on;
Verbindung Nr. 5: 4-{2-[4-(4-Chlor-3-trifluormethylphenyl)-3,6-dihydro-2*H*-pyridin-1-yl]-2-oxoethyl}-1-pyridin-2-ylpiperazin-2-on;
Verbindung Nr. 6: 4-{2-[4-(4-Chlorphenyl)-3,6-dihydro-2*H*-pyridin-1-yl]-2-oxoethyl}-1-pyridin-2-ylpiperazin-2-on;
Verbindung Nr. 7: 4-{2-[4-(2,3-Dichlorphenyl)-3,6-dihydro-2*H*-pyridin-1-yl]-2-oxoethyl}-1-(5-trifluormethylpyridin-2-yl)piperazin-2-on;
Verbindung Nr. 8: 4-{2-[4-(4-Chlorphenyl)-3,6-dihydro-2*H*-pyridin-1-yl]-2-oxoethyl}-1-(6-chlorpyridin-2-yl)piperazin-2-on;
Verbindung Nr. 9: 4-{2-[4-(3-Chlorphenyl)-3,6-dihydro-2*H*-pyridin-1-yl]-2-oxoethyl}-1-(5-trifluormethylpyridin-2-yl)piperazin-2-on;
Verbindung Nr. 10: 4-{2-[4-(4-Trifluormethylphenyl)-3,6-dihydro-2*H*-pyridin-1-yl]-2-oxoethyl}-1-(5-trifluormethylpyridin-2-yl)piperazin-2-on;
Verbindung Nr. 11: 4-{2-[4-(3-Trifluormethylphenyl)-3,6-dihydro-2*H*-pyridin-1-yl]-2-oxoethyl}-1-(5-trifluormethylpyridin-2-yl)piperazin-2-on;
Verbindung Nr. 12: 4-{2-[4-(4-Chlor-3-trifluormethylphenyl)-3,6-dihydro-2*H*-pyridin-1-yl]-2-oxoethyl}-1-pyridin-3-ylpiperazin-2-on;
Verbindung Nr. 13: 1-(6-Chlorpyridin-3-yl)-4-{2-[4-(4-chlor-3-trifluormethylphenyl)-3,6-dihydro-2*H-*pyridin-1-yl]-2-oxoethyl}piperazin-2-on;
Verbindung Nr. 14: 4-{2-Oxo-2-[5-(3-trifluormethylphenyl)-3,6-dihydro-2H-pyridin-1-yl]ethyl}-1-(5-trifluormethylpyridin-2-yl)piperazin-2-on;
Verbindung Nr. 15: 4-{2-Oxo-2-[4-(3-trifluormethoxylphenyl)-3,6-dihydro-2H-pyridin-1-yl]ethyl}-1-pyridin-2-ylpiperazin-2-on;
Verbindung Nr. 16: 4-{2-[4-(4-Chlor-3-trifluormethylphenyl)-2,5-dihydropyrrol-1-yl]-2-oxoethyl}-1-(5-trifluormethylpyridin-2-yl)piperazin-2-on;
Verbindung Nr. 17: 4-{2-[4-(3,5-Bistrifluormethylphenyl)-3,6-dihydro-2H-pyridin-1-yl]-2-oxoethyl}-1-(5-trifluormethylpyridin-2-yl)piperazin-2-on;
Verbindung Nr. 18: 4-{2-[4-(3-Methylphenyl)-3,6-dihydro-2H-pyridin-1-yl]-2-oxoethyl}-1-(5-trifluormethylpyiridin-2-yl)piperazin-2-on;
Verbindung Nr. 19: 4-{2-[4-Phenyl-3,6-dihydro-2H-pyridin-1-yl]-2-oxoethyl}-1-(5-trifluormethylpyridin-2-yl)piperazin-2-on;
Verbindung Nr. 20: 4-{2-Oxo-2-[5-(2,3-dichlorphenyl)-3,6-dihydro-2H-pyridin-1-yl]ethyl}-1-(5-trifluormethylpyridin-2-yl)piperazin-2-on;
Verbindung Nr. 21: 4-{2-Oxo-2-[5-(3-methoxyphenyl)-3,6-dihydro-2H-pyridin-1-yl]ethyl}-1-(5-trifluormethylpyridin-2-yl)piperazin-2-on;
als Basen oder als Säureadditionssalze.

10. Verfahren zur Herstellung einer Verbindung der Formel (I) nach einem der vorhergehenden Ansprüche, in der A, B, m, n, R3 wie zuvor definiert sind, umfassend den Schritt, dass man eine Verbindung der Formel (IV) 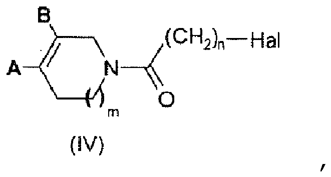 in der A, B, m und n wie in der allgemeinen Formel (I) definiert sind und Hal ein Halogenatom bedeutet,
mit einer Verbindung der allgemeinen Formel (V): 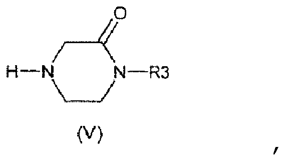 in der R3 wie in der allgemeinen Formel (I) definiert ist, in Gegenwart einer Base umsetzt.

11. Verbindung der Formel (IV), 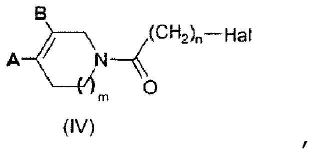 in der A, B, m und n wie in einem der Ansprüche 1 bis 9 definiert sind und Hal ein Halogenatom bedeutet;
mit Ausnahme von 2-Chlor-1-[4-(2-methoxyphenyl)-3,6-dihydro-2H-pyridin-1-yl]ethanon und 2-Chlor-1-[4-(4-bromphenyl)-3,6-dihydro-2H-pyridin-1-yl]ethanon; als Base oder als Säureadditionssalze.

12. Arzneimittel, **dadurch gekennzeichnet, dass** es eine Verbindung der Formel (I) nach einem der Ansprüche 1 bis 9 oder ein Additionssalz dieser Verbindung mit einer pharmazeutisch unbedenklichen Säure umfasst.

13. Pharmazeutische Zusammensetzung, **dadurch gekennzeichnet, dass** sie eine Verbindung der Formel (I) nach einem der Ansprüche 1 bis 9 oder ein pharmazeutisch unbedenkliches Salz sowie mindestens einen pharmazeutisch unbedenklichen Grundstoff umfasst.

14. Verbindung der allgemeinen Formel (I) nach einem der Ansprüche 1 bis 9 für die Herstellung eines Arzneimittels zur Vorbeugung oder Behandlung von neurodegenerativen Erkrankungen des zentralen und peripheren Nervensystems, seniler Demenz, Epilepsie, Morbus Alzheimer, Morbus Parkinson, Huntington-Chorea, Down-Syndrom, Prionenerkrankungen, Gedächtnisstörung, Schizophrenie, Depression, bipolarer affektiver Störung, amyotropher Lateralsklerose, Multipler Sklerose, Herz-Kreislauf-Störungen, postischämischen Herzschäden, Kardiomyopathien, Myokardinfarkt, Herzinsuffizienz, Herzischämie, Hirninfarkt; peripheren Neuropathien, Schäden des Nervus opticus und der Retina, Degeneration des Retinapigments, Glaukom, Retinaischämie, Makulardegeneration, Rückenmarkstraumen, Schädeltraumen, Arteriosklerose, Stenosen, Vernarbungsstörungen, Alopecia, Karzinomen, Tumoren, Metastasen, Leukämien, Atmungsstörungen, Lungenentzündung, Allergie, Asthma, chronischobstruktiver Atemwegserkrankung, Haut-, Körper-, Eingeweide- und Nervenschmerz, chronischen neuropathischen und entzündlichen Schmerzen, Autoimmunerkrankungen, rheumatoider Polyarthritis, ankylosierender Spondylitis, Psoriasis-Arthritis, Plaque-Psoriasis, Knochenbrüchen, Knochenerkrankungen, Osteoporose.

15. Verbindung nach Anspruch 14, die fähig ist, die Dimerisierung des P75^{NTR}-Rezeptors unabhängig von seinem Liganden zu hemmen.
